# EUROPEAN PATENT APPLICATION

(11) **EP 2 283 807 A1**
(43) Date of publication of application: **16.02.2011**
(21) Application number: 09742687.8
(22) Date of filing: 27.04.2009
(51) Int. Cl.: A61K 8/81, A61K 31/702, A61P 17/16, A61Q 19/00, A61Q 19/08

(54) **MOISTURIZING AGENT**

(30) Priority: 09.05.2008 JP 2008123637
(71) Applicant: Celagix, Res. Ltd., Kanagawa 2130002 (JP)
(72) Inventor: GOTO, Mitsuaki, Kawasaki-shi, Kanagawa 213-0002 (JP); IWAMA, Masamichi, Yokohama-shi, Kanagawa 229-0003 (JP); ANAN, Naoki, deceased (JP)
(74) Representative: Müller-Boré & Partner Patentanwälte
(86) International application number: PCT/JP2009/058239
(87) International publication number: WO 2009/136563

(57) **Abstract**

Disclosed is a moisturizing agent comprising a copolymer represented by formula (1). [In formula (1), A represents a substituent containing an oligosaccharide composed of 2 to 6 monosaccharide molecules bound to each other; B represents a group represented by formula (2); m and n represent numbers which meet the following equation m:n = 9:1 to 1:9; and Z represents a hydrogen group, a methyl group or an ethyl group.] [In formula (2), X represents a saturated or unsaturated monocyclic or dicyclic ring having 5 to 10 ring-constituting members, or a molecule containing a nitrogen or an oxygen, provided that the ring may contain 1 to 3 atoms independently selected from the group consisting of a nitrogen, a sulfur and an oxygen; n represents a number of 0 or 1; Y represents -NHCO-, -CONH-, -NHCONH-, -NH-, -CO, or an alkyl group having 0 or more carbon atoms; and R represents a linear or branched alkyl, alkenyl or alkynyl having 1 to 20 carbon atoms which, if required, may be substituted by one or two hydroxy, mercapto, amino, cyano, heterocyclyl, heterocyclyloxy, phenyl, benzyl, heteroaryl, phenoxy, benzyloxy or heteroaryloxy groups.]

## Description

### TECHNICAL FIELD

The present invention relates to a moisturizer comprising a copolymer composed of a sugar chain-containing styrene derivative and a monomer capable of copolymerizing with the sugar chain-containing styrene derivative, and a cosmetic comprising the moisturizer, a polysaccharide and a protein.

### BACKGROUND ART

Since naturally-occurring polysaccharides such as hyaluronic acid and naturally-occurring proteins such as collagen have superior moisturizing abilities, provide dampness and elasticity to skin, and have the effects of conditioning smoothness and providing suppleness, they are able to keep skin fine. Therefore, numerous highly moisturizing cosmetics comprising these components have been developed.

However, hyaluronic acid is poorly absorbed by skin because it is a highly viscous naturally-occurring polysaccharide, and hyaluronic acid is readily detached from the skin with water, sweat or the like. Thus, it had problems concerning the long-term moisturizing performance.

Furthermore, collagen used for cosmetics, the majority of which is obtained by extracting from animals or marine organisms and converting into low molecular weight products by treating with acid, alkali or enzyme, has almost no permeability to skin like hyaluronic acid. Thus, it could not be expected to have an effect of moisturizing inside skin, although its effect of moisturizing on the surface of skin is high.

Nevertheless, collagen is a major component protein that is in charge of the maintenance and activation of cells around the cells and, thus, decrease in or hardening of collagen around cells causes reduction in cell activity, decrease in cell number and loss of skin elasticity, and serves as one of causes of wrinkle formation. Thus, for preventing wrinkle formation, it is important to promote the maintenance of collagen as well as the maintenance and production of hyaluronic acid, and to keep moisture inside and on the surface of skin cells by supplying collagen to cells in skin, in particular in dermal layer.

As described above, it was difficult to supply hyaluronic acid or collagen to skin cells only by including such a compound as a component as conventionally conducted, resulting in insufficient provision of a moisturizing property and suppression of wrinkle formation.

Poly(N-p-vinylbenzyl-D-lactoneamide) (hereinafter referred to as PVLA), which has a sugar as the side chain and styrene as the main chain, was synthesized as a saccharide having some modification being added (Non-Patent Document 1). There are cases where the macromolecule is used as a substrate for cell culture (Patent Documents 1 to 4), as a coating agent for culture vessel (Patent Document 5), as an antithrombotic material (Patent Document 6), for a DDS by including a drug with PVLA (Patent Document 7), for a cosmetic (Patent Document 8), etc.. PVLA is used as a homopolymer in all of these cases.

On the other hand, there are a method for incorporating a drug into specific cells using a copolymer with a lysine oligomer having a methacryloyl group on the N terminus as a copolymer of this sugar chain-containing styrene derivative and another monomer (Patent Document 9), a case where a copolymer with 4-vinylbenzyl hexadecaneamide is used as an antithrombotic material (Patent Document 10), etc.

The inventions in which such sugar chain-containing polymers are used utilize the specific effects of the sugar side chains in cell culture, the recognition of the sugar side chains by cells, etc. The use of such a copolymer as a moisturizer has not been known.

### PRIOR ART DOCUMENTS

### PATENT DOCUMENTS

Patent Document 1: Japanese Laid-open Patent [Kokai] Publication No. Sho 63-116692
Patent Document 2: Japanese Laid-open Patent [Kokai] Publication No. Sho 63-279787
Patent Document 3: Japanese Laid-open Patent [Kokai] Publication No. Sho 63-301786
Patent Document 4: Japanese Laid-open Patent [Kokai] Publication No. Hei 1-309681
Patent Document 5: Japanese Laid-open Patent [Kokai] Publication No. Hei 4-169530
Patent Document 6: Japanese Laid-open Patent [Kokai] Publication No. Hei 2-224664
Patent Document 7: Japanese Laid-open Patent [Kokai] Publication No. Hei 5-105637
Patent Document 8: Japanese Laid-open Patent [Kokai] Publication No. 2000-273019
Patent Document 9: Japanese Laid-open Patent [Kokai] Publication No. Hei 10-45630
Patent Document 10: Japanese Laid-open Patent [Kokai] Publication No. 2005-112987

### NON-PATENT DOCUMENTS

Non-Patent Document 1: K. Kobayashi, et al., Polymer Journal, Vol. 17, No. 4, pp. 567-575 (1985)

### SUMMARY OF INVENTION

### Problems to be Solved by the Invention

An object of the present invention is to improve a moisturizing property which is a conventional problem, and to provide a cosmetic that has high permeability into skin, and is effective in suppressing wrinkle formation.

### Means to Solve the Problems

As a result of intensive studies for solving the above-mentioned problems, the present inventors have found that a copolymer composed of a sugar chain-containing styrene derivative and a monomer capable of copolymerizing with the sugar chain-containing styrene derivative has very high moisturizing performance, and that a cosmetic comprising said copolymer, and a polysaccharide and/or a protein suppresses wrinkle formation, thereby completed the present invention.

Thus, the present invention is as described below.
1. A moisturizer comprising a copolymer of formula (1): 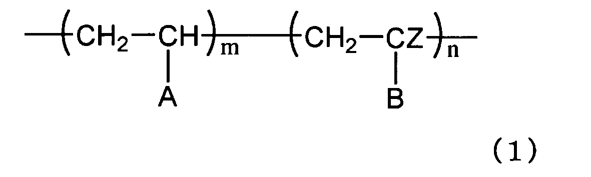 [wherein
   A is a substituent containing an oligosaccharide in which 2 to 6 monosaccharide molecules are bound to each other;
   B is a group of formula (2): 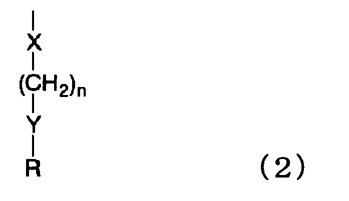
   (wherein X represents a 5- to 10-membered, saturated or unsaturated, monocyclic or dicyclic ring, or nitrogen- or oxygen-containing molecule, wherein the ring may contain 1 to 3 atom(s) selected from the group consisting of nitrogen, sulfur and oxygen; n is 0 or 1; Y is -NHCO-, -CONH-, -NHCONH-, -NH- or -CO-, or an alkyl group of 0 or more carbon(s); R is a linear or branched alkyl, alkenyl or alkynyl of 1 to 20 carbon(s), which may optionally be substituted with one or two hydroxy, mercapto, amino, cyano, heterocyclyl, heterocyclyloxy, phenyl, benzyl, heteroaryl, phenoxy, benzyloxy or heteroaryloxy);
   m:n = 9:1 to 1:9;
   Z is a hydrogen group, methyl group or ethyl group];
2. A moisturizer comprising one or more copolymer(s) selected from the group consisting of:
   a copolymer of formula (3): 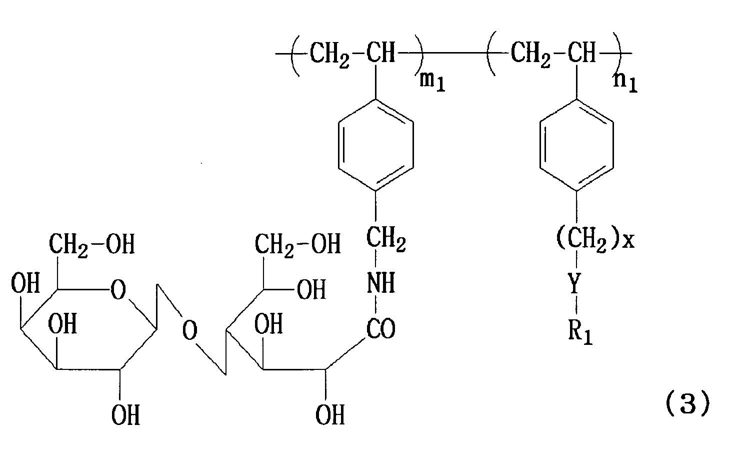 [wherein
   m₁:n₁ is 9:1 to 1:9;
   x is an integer of 0 to 3;
   Y is a single bond, -NHCO-, -NHCONH-, -CONH-, -OCONH-, -NHCOO-, -OCO-, -COO-, -CO- or -NH-;
   R₁ is a saturated or unsaturated, monocyclic or dicyclic ring of 5 to 10 carbon(s), or a 5-or 6-membered heterocyclic ring containing O, S or N (wherein the monocyclic or dicyclic ring, or the heterocyclic ring may be unsubstituted or substituted with one or more group(s) selected from the group consisting of halogen, hydroxy, amino, thiol, cyano, cyclopentyl, cyclohexyl, phenyl and acetyl), or alkyl of 1 to 20 carbon(s) (wherein the alkyl may be interrupted with O, S or NH, and may be substituted with one or more group(s) selected from the group consisting of halogen, hydroxy, amino, thiol, cyano, cyclopentyl, cyclohexyl, phenyl and acetyl)],
   a copolymer of formula (4): 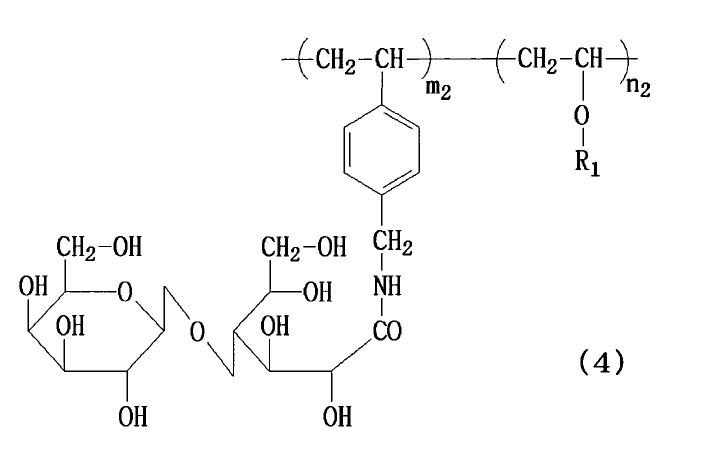 (wherein m₂:n₂ = 9:1 to 1:9; R₁ is as defined for formula (3)); and
   a copolymer of formula (5): 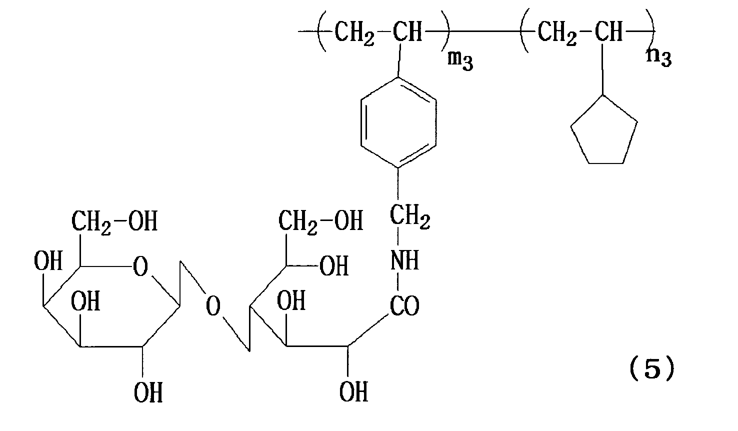 (wherein m₃:n₃ = 9:1 to 1:9);
3. The moisturizer according to 2 above, wherein Y is NHCO- or -CONH-, and R₁ is alkyl of 1 to 20 carbon(s) (wherein the alkyl is substituted with halogen, hydroxy or cyclohexyl);
4. The moisturizer according to any one of 1 to 3 above, wherein the content of the copolymer is 0.0001 to 5% by weight;
5. A cosmetic comprising the moisturizer according to any one of 1 to 3 above, and a polysaccharide and/or a protein;
6. The cosmetic according to 5 above, wherein the polysaccharide is chondroitin;
7. The cosmetic according to 5 above, wherein the polysaccharide is hyaluronic acid;
8. The cosmetic according to 5 above, wherein the protein is collagen or low-molecular-weight collagen;

### Effects of the Invention

As described above, the present invention is a moisturizer comprising a copolymer composed of a sugar chain-containing styrene derivative and a monomer capable of copolymerizing with the sugar chain-containing styrene derivative, and a cosmetic comprising the moisturizer, a polysaccharide and a protein. The cosmetic of the present invention suppresses wrinkle formation and has very high moisturizing performance.

### BRIEF DESCRIPTION OF THE DRAWINGS

FIG. 1 shows the results of cell growth tests for SRA cells on Petri dishes coated with various polymers. From left to right, 0.01% PVLA, 0.01% P(VLA-co-VBCH) 9:1, 0.01% PVCA, 0.01% P(VCA-co-VBCH) 9:1, control (water only). The values indicate the ratio of growth defining the value 24 hours after Pst as 100%.
FIG. 2 shows the results of cell growth tests for NIH3T3 cells on Petri dishes coated with various polymers. From left to right, 0.01% PVLA, 0.01% P(VLA-co-VBCH) 9:1, 0.01% PVCA, 0.01% P(VCA-co-VBCH) 9:1, control (water only). The values indicate the ratio of growth defming the value 24 hours after Pst as 100%.
FIG. 3 shows the results of pig skin water permeation tests (after 15 minutes).
FIG. 4 shows the results of pig skin water permeation tests (after 30 minutes).
FIG. 5 shows the results of pig skin water permeation tests (after 60 minutes).

### MODE FOR CARRYING OUT THE INVENTION

The best mode for carrying out the present invention is explained in detail below.

Although lactose is used as a sugar in the compounds of the above-mentioned formulas, in another aspect of the present invention, other oligosaccharides in which 2 to 6 monosaccharide molecules are bound to each other can be used preferably. Specifically, it is preferably one selected from the group consisting of trehalose, neotrehalose, maltose, cellobiose, gentiobiose, melibiose, rutinose, sucrose, turanose, vicianose, cellotriose, gentianose, isopanose, maltotriose, raffinose, lycotetraose, maltotetraose, maltopentaose and maltohexaose. Among these, lactose is particularly preferable from the viewpoints of solubility of the polymer in water, cost of the raw material, readiness of obtaining, and the like. In addition, the monosaccharide is preferably one selected from the group consisting of glucose, galactose, mannose, fructose, xylose and arabinose. Among these, glucose is particularly preferable for the same reasons as the above.

The moisturizer according to the present invention comprises a copolymer of any one of the above-mentioned formulas 1 to 3. Furthermore, the cosmetic according to the present invention comprises, in addition to the moisturizer of the present invention, a polysaccharide such as sodium hyaluronate or chondroitin, and/or a protein such as low-molecular-weight collagen or collagen.

Sodium hyaluronate is industrially produced by an extraction method in which it is extracted from an animal tissue or the like, or a fermentation method in which it is obtained by culturing a microorganism. Sodium hyaluronate that can be used in the present invention may be produced by either the extraction method or the fermentation method.

Furthermore, sodium hyaluronate having a molecular weight of about 50,000 to 3,000,000 is produced. Sodium hyaluronate having any molecular weight within this range can be used in the present invention.

Atelocollagen, which is obtained by extraction and purification from skin or the like of an animal such as cow, pig or chicken, or fish or shellfish, and solubilization by allowing an enzyme to act for increasing the solubility in water, can preferably be used as collagen in the present invention. Since the solubility is excellent also in the weakly acidic to alkaline range, atelocollagen having chemical modification such as myristylation, phthalylation or succinylation can be preferably used. Furthermore, use in combination with sodium hyaluronate is particularly preferable because an excellent moisturizing action is exerted therewith.

As to the low-molecular-weight collagen used in the present invention, a collagen peptide having a molecular weight of about 280 to 1000, which is obtained by converting into low molecular weight products by enzymatic treatment of a collagen component or gelatin component extracted and purified from skin or the like of an animal such as cow, pig or chicken, or fish or shellfish, has skin permeability and high retention properties in dermal layer, and is highly effective in promoting the production of collagen and hyaluronic acid. A collagen peptide having a molecular weight of 1000 or more is not preferable because it is inferior in skin permeability.

The copolymer of the present invention has a basic backbone in which a sugar chain is bound to a hydrophobic polymer. Therefore, it prevents evaporation of water from the surface of skin, has an action of retaining water inside skin, and has an extremely high moisturizing effect on skin. In particular, use in combination with a skin-permeating collagen peptide or hyaluronic acid results in an excellent moisturizing effect on the surface of skin and inside skin and is very effective in inhibiting the formation of wrinkles on skin due to the combined action by them.

As to the ratio of the amount of component (a), sodium hyaluronate, and component (b), sum of low-molecular-weight collagen and collagen to be mixed, preferably (a)/{(b)+(c)} = 10 to 220 (by weight) (wherein (c) is another added component), more preferably 30 to 200 (by weight). Beyond this range, the homogeneity, the moisturizing property and the spreading upon use are reduced.

As to the ratio of collagen to be mixed with the copolymer (d) of the present invention, preferably (b)/(d) = 0.07 to 1.2 (by weight), more preferably 0.1 to 0.8 (by weight). Beyond this range, the moisturizing property and the effect of suppressing skin wrinkle formation are reduced.

The cosmetic of the present invention may comprise an arbitrary component other than the components of the present invention as long as it is in a qualitative and quantitative range that does not impair the action and effect of the present invention. For example, it can comprise an amino acid, a sugar, a vitamin C derivative, a thickener, a flavoring agent, a preservative or the like.

Although the present invention is explained more specifically with Examples below, the present invention is not limited thereto.

The copolymers of the present invention were produced as follows.
Polystyrene derivatives having sugar chains including VLA (N-p-vinylbenzyl-O-β-D-galactopyranosyl-(1-4)-D-gluconamide) were synthesized according to Patent Document 10.
The synthesis of a monomer, vinylbenzyl hexadecaneamide (VAL, the compound of formula (3) wherein X = 1, Y = NHCO, R1 = -(CH₂)₁₄CH₃), is described. 10 g of vinylbenzylamine was dissolved in 150 ml of anhydrous chloroform, 7 g of pyridine was added, and the mixture was cooled. Then, 24 g of palmitoyl chloride dissolved in 50 ml of anhydrous chloroform was added dropwise over 30 minutes using a dropping funnel. After completion of the reaction, the chloroform solution was washed with water and dried, and the solvent was distilled off under reduced pressure. The residue was recrystallized from ethanol to obtain 20 g of VAL.

For synthesizing a polymer, VLA and VAL were weighed at a molar ratio of 9:1, 8:2, 7:3 or the like, and the mixture was dispensed into vacuum reaction tubes. After complete dissolution by addition of DMSO or a DMSO-toluene mixed solvent, a freezing-degassing-thawing cycle was repeated to remove oxygen from the solution. Azoisobutyronitrile (AIBN) was added thereto at a molar ratio of 1/100 and dissolved, and the tubes were then sealed under reduced pressure. The polymer was synthesized by reacting for 5 hours at 60°C.

The resulting solution was added dropwise into an excess of methanol to precipitate the polymer. The procedure of dissolving the precipitate in an appropriate solvent and adding the solution dropwise into methanol again to precipitate the polymer was repeated three times.
The finally obtained polymer was dissolved in distilled water, dialyzed against an excess of water, and then lyophilized to obtain the polymer of interest.

Next, synthesis of vinylbenzylcyclohexylpropaneamide (VBCH, the compound of formula (3) wherein X = 1, Y = NHCO and R1 = 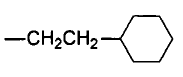 ), which is one of the partner monomers for copolymerization of VLA, is described. 13 g of vinylbenzylamine was dissolved in 150 ml of anhydrous THF, and 23 g of WSC and 13 g of 3-cyclohexylpropionic acid dissolved in 50 ml of anhydrous THF were added. After completion of the reaction, the precipitate was filtered out, and THF was distilled off. After dissolving in chloroform, the solution was washed with water and then dried, and the solvent was distilled off under reduced pressure. The residue was recrystallized from ethanol to obtain 10 g of the monomer. The polymer was produced in the same manner as that described above.

As to other monomers, commercially available products were purchased.

### Cell Culture Test

A 0.01 % (w/v) aqueous solution of P(VLA-co-VAL) 9:1 or P(VCA-co-VAL) 9:1 was prepared, 2-ml aliquots were added to an untreated 6-well plate for coating for one whole day. Then, the plate was washed three times with PBS and used for experiments.

### Experimental Method

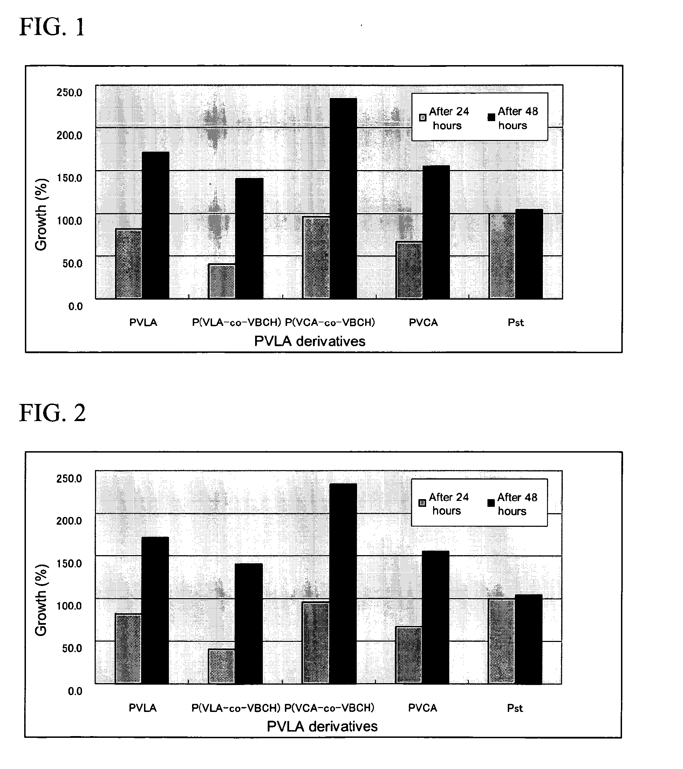

SRA (lens epithelial) cells and 3T3 (fibroblast) cells which had been passaged were seeded into the plate (1 x 10⁴ cells/plate). Culture media optimal for the respective cells were used. The morphology was observed and the cell number was counted after culturing for 24 hours or 48 hours. The results are shown in FIG. 1 and FIG. 2.

PVLA has a galactose residue, while PVCA has a cellobiose residue. As a sugar chain, it was found that the cellobiose residue was recognized well by cells to promote the growth. The obtained results show that each derivative to which a hydrophobic anchor was introduced promoted the cell growth more efficiently. Thus, it is considered that use of the copolymer having a sugar chain produced according to the present invention is effective in activating cells to promote the growth.

### Contact Angle Test

1-ml aliquots of one of test solutions were added to polystyrene Petri dishes (Falcon 1008 Petri Dish, no surface treatment) and the dished were allowed to stand for 1 hour to coat the surfaces of dishes. After a prescribed period of time, each dish was washed three times with 3 ml of distilled and dried once. The contact angles of water drops dropped onto the dishes were measured for the dried dishes using a contact angle measurement apparatus.

**Table 1**

| Contact angle on the surface of Petri dish coated with solution | |
|---|---|
| Sample No. | Contact Angle |
| (1) 0.1 % Hyaluronic acid solution | 87.4 |
| (2) 0.1% Chondroitin solution | 89.6 |
| (3) 0.1% P(VLA-co-VBCH) 9:1 solution | 56.6 |
| (4) Mixed solution of 0.1% hyaluronic acid and 0.1% P(VLA-co-VBCH) 9:1 | 36 |
| (5) Mixed solution of 0.1% chondroitin and 0.1% P(VLA-co-VBCH) 9:1 | 61.1 |
| (6) Control (water only) | 83.6 |
| (7) 0.1 % PVLA | 50.7 |
| (8) 0.1% P(VLA-co-VAL) 9:1 solution | 59.4 |
| (9) Mixed solution of 0.1% hyaluronic acid and 0.1% P(VLA-co-VAL) 9:1 | 58.8 |
| (10) Mixed solution of 0.1% chondroitin and 0.1% P(VLA-co-VAL) 9:1 | 67 |
| (11) Mixed solution of 0.1% hyaluronic acid and 0.1 % PVLA | 62.7 |
| (12) Mixed solution of 0.1% chondroitin and 0.1% PVLA | 65.1 |

As indicated in the table above, high water repellency of 83.6 degrees was observed for the control (water only). The contact angles for the solutions to which only hyaluronic acid or chondroitin sulfate was added tended to be more water-repellent (87.4 and 89.6 degrees, respectively), demonstrating that they are not suitable for making a polystyrene surface hydrophilic.

On the other hand, it was found that the solution containing the synthetic sugar chain polymer could provide a more hydrophilic surface than the water system. As to the systems of the combinations of hyaluronic acid and a compound in which a hydrophobic residue was copolymerized with PVLA, which is characteristic of the present case, it was found that they exhibit very great abilities to make hydrophilic of 36 degrees for the system of 0.1% P(VLA-co-VBCH) 9:1 and hyaluronic acid, and 58.8 degrees for the system of the mixed solution of hyaluronic acid and 0.1% P(VLA-co-VAL) 9:1. As to the combinations with chondroitin sulfate, satisfactory abilities to make hydrophilic were observed as well although the effects were less than those with hyaluronic acid.

Thus, based on these results, it was found that a surface being made very hydrophilic can be provided due to the anchoring effect of the hydrophobic residue of PVLA derivative and the fixing effect of the polysaccharide component such as hyaluronic acid.

### Hyaluronic Acid Moisture Retention Test

Moisture retention tests were carried out using hyaluronic acid and PVLA derivatives.
1-ml aliquots of each sample were dispensed into sample bottles (5-ml Maruemu Screw Bottles). The bottles together with sample bottle caps were weighed. In a sealed container, the samples were arranged placing a sample bottle containing 10 g of calcium chloride in the center, the caps were removed, and the samples were allowed to stand. The weights were then measured after a prescribed period of time.

The results are shown below.

**Table 2**

| Sample | Weight on day 20 defining the value for water as 0 | |
|---|---|---|
| 0.1 % Hyaluronic acid solution | 974.8 | 49.4 |
| 0.1% Chondroitin solution | 977.4 | 52.0 |
| 0.1% P(VLA-co-VBCH) 9:1 solution (Lot. 070613-2) | 985.0 | 59.6 |
| Mixed solution of 0.1% hyaluronic acid and 0.1% P(VLA-co-VBCH) 9:1 | 998.2 | 72.8 |
| Mixed solution of 0.1% chondroitin and 0.1% P(VLA-co-VBCH) 9:1 | 984.8 | 59.4 |
| Control (water only) | 925.4 | -0.1 |
| 0.1 % PVLA solution (Lot. 050721) | 933.0 | 7.5 |
| 0.1 % P(VLA-co-VAL) 9:1 solution (Lot. none) | 938.2 | 12.8 |
| Mixed solution of 0.1% hyaluronic acid and 0.1% P(VLA-co-VAL) 9:1 | 945.6 | 20.1 |
| Mixed solution of 0.1% chondroitin and 0.1% P(VLA-co-VAL) 9:1 | 934.3 | 8.9 |

As a result, it was found that the respective samples retained more moisture than water as the control on day 20. Although this effect was remarkable even for the sugar chain macromolecule alone, it was found that the effect became more remarkable by combining the sugar chain macromolecule with the polysaccharide.

In addition, the effect was further increased when the hydrophobic copolymer was introduced into the sugar chain macromolecule. This shows that the hydrophobic copolymer plays an important role in the interactions between the sugar chain macromolecule and the polysaccharide. In particular, the effect was more remarkable for the sugar chain macromolecule having a cyclohexane ring being introduced. Thus, it is considered that evaporation of water is prevented and moisture is effectively retained by adopting a rigid cluster structure among the sugar chain macromolecule, the polysaccharide and water.

### Skin Moisture Retention Test

Moisture levels on arms of volunteers were measured using a commercially available skin moisture measurement apparatus. 100-µl aliquots of each aqueous solution were applied to measured skin surfaces, which were then covered with cotton for a prescribed period of time. After the prescribed period of time, the cotton was removed and the skin moisture level at each test site was measured. The results of measurement of moisture levels after 30 minutes are shown below. The values represent the differences in skin moisture levels before and after treatment defining the value for the difference in moisture levels when water was applied as 0.

**Table 3**

| | | | | | |
|---|---|---|---|---|---|
| 0.1% hyaluronic acid solution | -1.6 | 7.2 | 2.4 | 4.5 | 0.3 |
| 0.1% chondroitin solution | -0.4 | -0.5 | 6.6 | 2.2 | -0.8 |
| 0.1 % P(VLA-co-VBCH) 9:1 solution | 0 | -3 | 0.5 | 3.2 | 1.3 |
| Mixed solution of 0.1% hyaluronic acid and 0.1% P(VLA-co-VBCH) 9:1 | 15.5 | 11.2 | 10.6 | 5.1 | 3.5 |
| Mixed solution of 0.1% chondroitin and 0.1 % P(VLA-co-VBCH) 9:1 | 14.4 | -0.1 | 13.4 | 4.9 | 1.7 |
| Control (water only) | 0 | 0 | 0 | 0 | 0 |
| 0.1% PVLA | -1.2 | 2.2 | -1.4 | 3.3 | 0.2 |
| 0.1% P(VLA-co-VAL) 9:1 solution | 0.6 | 2.4 | 5.6 | 4 | 0.3 |
| Mixed solution of 0.1% hyaluronic acid and 0.1% P(VLA-co-VAL) 9:1 | 2.5 | 4.1 | 19.6 | 3.4 | 2.1 |
| Mixed solution of 0.1% chondroitin and 0.1% P(VLA-co-VAL) 9:1 | 13 | 0.4 | 10 | 0.1 | -0.1 |
| Mixed solution of 0.1% hyaluronic acid and 0.1 % PVLA | 0.8 | 11.5 | 22.1 | 3.2 | 1.9 |
| Mixed solution of 0.1% chondroitin and 0.1% PVLA | 2.2 | -1.8 | 12 | 2.4 | -3.5 |

As a result of total comparison, effects of retaining moisture were observed even for the sugar chain-containing polymer alone. Furthermore, dramatic moisture retention effects were obtained for the systems in which the sugar chain macromolecule was combined with a polysaccharide such as hyaluronic acid or chondroitin sulfate. This effect was higher for the systems in which a hydrophobic residue was introduced into the sugar chain macromolecule. Thus, it is considered that introduction of the hydrophobic copolymer into the sugar chain macromolecule can enhance the interaction with skin and allow the polysaccharides to stably remain on the skin by anchoring to the skin. This effect was observed even after one hour.

### Pig Skin Water Permeation Test

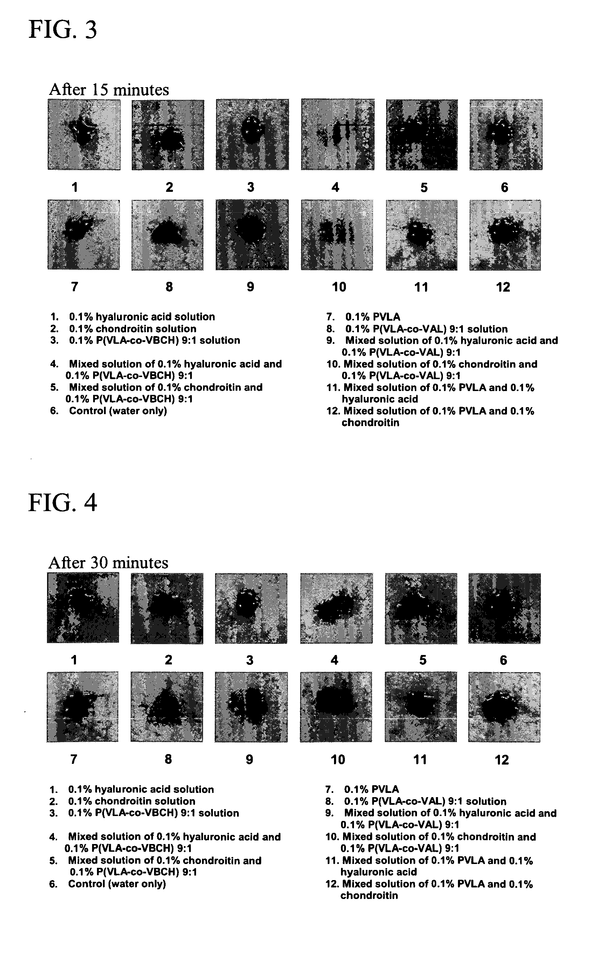

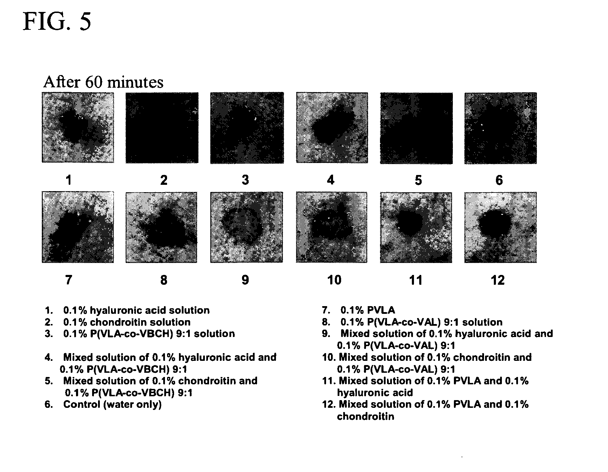

A trace amount of a trypan blue solution was added to each aqueous solution to prepare a test solution. Pig skin was prepared and cut into 3-cm squares. 30-µl aliquots of the aqueous solution were applied to the surfaces of the pig skins and allowed to stand for a prescribed period of time. After the prescribed period of time, photographs of the respective test sites were taken using a digital camera for comparison. The measurement results after varying periods of time are shown in FIGs. 3 to 5.

The respective solutions tended to be absorbed by the skin well when compared with the photograph showing the case where water was applied. In particular, the effect was remarkable for the polymers into which a hydrophobic moiety was introduced, although a tendency to readily penetrate into skin was observed even for the sugar chain-containing polymer alone.

Furthermore, dramatic skin permeability was obtained for the systems in which the sugar chain polymer was combined with a polysaccharide such as hyaluronic acid or chondroitin sulfate. This effect was higher for the systems in which a hydrophobic residue was introduced into the sugar chain macromolecule. Thus, it is considered that introduction of the hydrophobic copolymer into the sugar chain macromolecule can enhance the interaction with skin and allow the polysaccharide to stably remain on the skin by anchoring to the skin.

This effect was observed even after one hour. In addition, skin permeation in the system of water alone was slow even after one hour and water was floating. Thus, the skin water permeation action of the sugar chain polymer was very effective.

Also in cases where other sugar chains were used, water solubility was retained, polystyrene dishes could readily be coated, the coated surfaces were made hydrophilic, and contact angles similar to those in the cases of PVLA derivatives were observed. Thus, similar effects were obtained also with PVCA and PVMA.

### Milky Lotion

Milky lotions of the following compositions were prepared according to the following production method, and the effects of beautifying skin and the effects of preventing skin aging were examined.

**Table 4**

| | Product of Present Invention | Comparative Products | |
|---|---|---|---|
| (1) Squalane | 5.0 | 5.0 | 5.0 |
| (2) Vaseline | 2.0 | 2.0 | 2.0 |
| (3) Beeswax | 0.5 | 0.5 | 0.5 |
| (4) Sorbitan sesquioleic acid ester | 0.8 | 0.8 | 0.8 |
| (5) Polyoxyethyleneoleyl ether | 1.2 | 1.2 | 1.2 |
| (6) 1,3-Butylene glycol | 5.0 | 5.0 | 5.0 |
| (7) PVLA derivative (P(VLA-co-VBCH) 9:1) | 0.1 | 0.05 | 0 |
| (8) dl-α-Tocopherol | 0.01 | 0.01 | 0.01 |
| (9) Ethyl alcohol | 5.0 | 5.0 | 5.0 |
| (10) Preservative | 0.2 | 0.2 | 0.2 |
| (11) Flavoring agent | 0.1 | 0.1 | 0.1 |
| (12) Xanthan gum (2% aqueous solution) | 20.0 | 20.0 | 20.0 |
| (13) Hyaluronic acid | 0.1 | 0.1 | 0.1 |
| (14) Purified water | q.s. | q.s. | q.s. |

A. Components 7, 9 and 14 were mixed while heating and kept at 70°C.
B. Components 1 to 6, 8, 10 and 13 were mixed while heating and kept at 70°C.
C. The mixture of B was added to the mixture of A and mixed, component 12 was added, and the mixture was emulsified to homogeneity. After cooling to 30°C, component 11 was added, and the mixture was mixed to homogeneity to obtain a milky lotion.

The test milky lotion was applied to the faces of a total of 20 women at the age of 30 to 55 years twice a day in the morning and at night after washing the faces for 20 weeks. The effects of beautifying skin and the effects of preventing skin aging due to the application were judged according to the following criteria.

### Effect of beautifying skin

| [Evaluation] | [Description] |
|---|---|
| Effective | Dull skin tone no longer conspicuous |
| Slightly effective | Dull skin tone not so conspicuous |
| Ineffective | No change as compared with before use |

### Effect of preventing skin aging

| [Evaluation] | [Description] |
|---|---|
| Effective | Suppleness and glossiness of skin improved |
| Slightly effective | Suppleness and glossiness of skin slightly improved |
| Ineffective | No change as compared with before use |

**Table 5**

| Test Milky Lotion | Effect of Beautifying Skin | | | Effect of Preventing Skin Aging | | |
|---|---|---|---|---|---|---|
| | Effective | Slightly Effective | Ineffective | Effective | Slightly Effective | Ineffective |
| Product of Present Invention | 16 | 4 | 0 | 13 | 7 | 0 |
| Comparative Product 1 | 1 | 9 | 10 | 3 | 7 | 10 |
| Comparative Product 2 | 1 | 10 | 9 | 3 | 7 | 10 |

### Cream

Creams of the following compositions were prepared according to the following production method, and the effects of beautifying skin were examined.

**Table 6**

| | Product of Present Invention | Comparative Products | |
|---|---|---|---|
| (1) Beeswax | 6.0 | 6.0 | 6.0 |
| (2) Cetanol | 5.0 | 5.0 | 5.0 |
| (3) Reduced lanolin | 5.0 | 5.0 | 5.0 |
| (4) Squalane | 30.0 | 30.0 | 30.0 |
| (5) Glycerin monoacetate | 4.0 | 4.0 | 4.0 |
| (6) Lipophilic glyceryl monostearate | 2.0 | 2.0 | 2.0 |
| (7) Polyoxyethylene sorbitan monolauric acid ester | 2.0 | 2.0 | 2.0 |
| (8) PVLA derivative (P(VLA-co-VBCH) 9:1) | 0.1 | 0.1 | 0 |
| (9) Mannitol | 1.0 | 1.0 | 1.0 |
| (10) Preservative | 0.2 | 0.2 | 0.2 |
| (11) Flavoring agent | 0.05 | 0.05 | 0.05 |
| (12) Hyaluronic acid | 0.1 | 0 | 0.1 |
| (13) Purified water | q.s. | q.s. | q.s. |

A. Components 1 to 8, 10 and 11 were mixed, heated and kept at 70°C.
B. Component 13 was heated and kept at 70°C.
C. The component of B was added to the mixture of A and mixed. After cooling, components 9 and 12 were added, and the mixture was mixing to homogeneity to obtain a cream.

The test cream was applied to the faces of a total of 20 women at the age 30 to 55 years twice a day in the morning and at night after washing the faces for 20 weeks. The effects of beautifying skin and the effects of preventing skin aging due to the application were judged according to the following criteria.

### Effect of beautifying skin

| [Evaluation] | [Description] |
|---|---|
| Effective | Dull skin tone no longer conspicuous |
| Slightly effective | Dull skin tone not so conspicuous |
| Ineffective | No change as compared with before use |

### Effect of preventing skin aging

| [Evaluation] | [Description] |
|---|---|
| Effective | Suppleness and glossiness of skin improved |
| Slightly effective | Suppleness and glossiness of skin slightly improved |
| Ineffective | No change as compared with before use |

**Table 7**

| Test Cream | Effect of Beautifying Skin | | | Effect of Preventing Skin Aging | | |
|---|---|---|---|---|---|---|
| | Effective | Slightly Effective | Ineffective | Effective | Slightly Effective | Ineffective |
| Product of Present Invention | 12 | 8 | 0 | 10 | 5 | 5 |
| Comparative Product I | 1 | 12 | 7 | 3 | 12 | 5 |
| Comparative Product 2 | 3 | 7 | 9 | 2 | 7 | 11 |

### Beauty Wash

| (Formulation) | Amounts (%) |
|---|---|
| (1) Glycerin | 5.0 |
| (2) 1,3-butylene glycol | 6.5 |
| (3) Polyoxyethylene sorbitan monolauric acid ester (20 E.O.) | 1.2 |
| (4) Ethyl alcohol | 8.0 |
| (5) Astaxanthin | 0.0005 |
| (6) SOD *1 | 0.5 |
| (7) PVLA derivative | 0.05 |
| (8) Preservative | q.s. |
| (9) Flavoring agent | q.s. |
| (10) Purified water | q.s. |

| | |
|---|---|
| *1: Sigma, obtained from human erythrocytes (5,230 units/mg) | |

A. Components (3), (4), (5), (7), (8) and (9) were mixed and dissolved.
B. Components (1), (2), (6) and (10) were mixed and dissolved.
C. The mixtures of A and B were mixed to homogeneity to obtain a beauty wash.

### Pack:

| (Formulation) | Amounts (%) |
|---|---|
| (1) Polyvinyl alcohol | 20.0 |
| (2) Ethyl alcohol | 20.0 |
| (3) Glycerin | 5.0 |
| (4) Kaolin | 6.0 |
| (5) Astaxanthin | 0.05 |
| (6) PVLA derivative | 0.1 |
| (7) Preservative | 0.2 |
| (8) Flavoring agent | 0.1 |
| (9) Purified water | q.s. |

A. Components (1), (3), (4) and (9) were mixed, heated to 70°C and stirred.
B. Components (2), (5), (7) and (8) were mixed.
C. The mixture of B was added to the mixture of A and mixed. After cooling, component (6) was dispersed to homogeneity to obtain a pack.

### Cleanser

| (Formulation) | Amounts (%) |
|---|---|
| (1) Stearic acid | 10.0 |
| (2) Palmitic acid | 8.0 |
| (3) Myristic acid | 12.0 |
| (4) Lauric acid | 4.0 |
| (5) Oleyl alcohol | 1.5 |
| (6) Purified lanolin | 1.0 |
| (7) Flavoring agent | 0.1 |
| (8) Preservative | 0.2 |
| (9) Glycerin | 18.0 |
| (10) Potassium hydroxide | 6.0 |
| (11) Astaxanthin | 0.5 |
| (12) dl-α-tocopherol acetate *1 | 0.05 |
| (13) PVLA derivative | 0.1 |
| (14) Purified water | q.s. |

| | |
|---|---|
| *1: Sigma | |

A. Components (9), (10) and (14) were mixed and heated to 70°C.
B. Components (1) to (6), (8), (11) and (12) were mixed and heated to 70°C.
C. The mixture of B was added to the mixture of A and kept at 70°C for a short while. After completion of the saponification reaction, the mixture was cooled to 50°C, components (7) and (13) were added, and the mixture was cooled to obtain a cleanser.

## Claims

1. A moisturizer comprising a copolymer of formula (1): 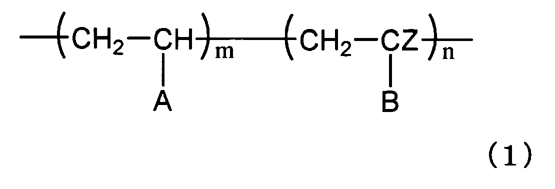 [wherein
A is a substituent containing an oligosaccharide in which 2 to 6 monosaccharide molecules are bound to each other;
B is a group of formula (2): 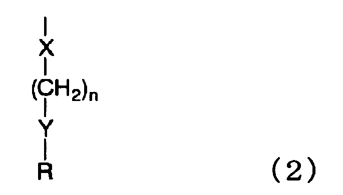
(wherein X represents a 5- to 10-membered, saturated or unsaturated, monocyclic or dicyclic ring, or nitrogen- or oxygen-containing molecule, wherein the ring may contain 1 to 3 atom(s) selected from the group consisting of nitrogen, sulfur and oxygen; n is 0 or 1; Y is -NHCO-, -CONH-, -NHCONH-, -NH- or -CO-, or an alkyl group of 0 or more carbon(s); R is a linear or branched alkyl, alkenyl or alkynyl of 1 to 20 carbon(s), which may optionally be substituted with one or two hydroxy, mercapto, amino, cyano, heterocyclyl, heterocyclyloxy, phenyl, benzyl, heteroaryl, phenoxy, benzyloxy or heteroaryloxy);
m:n = 9:1 to 1:9;
Z is a hydrogen group, methyl group or ethyl group].

2. A moisturizer comprising one or more copolymer(s) selected from the group consisting of:
a copolymer of formula (3): 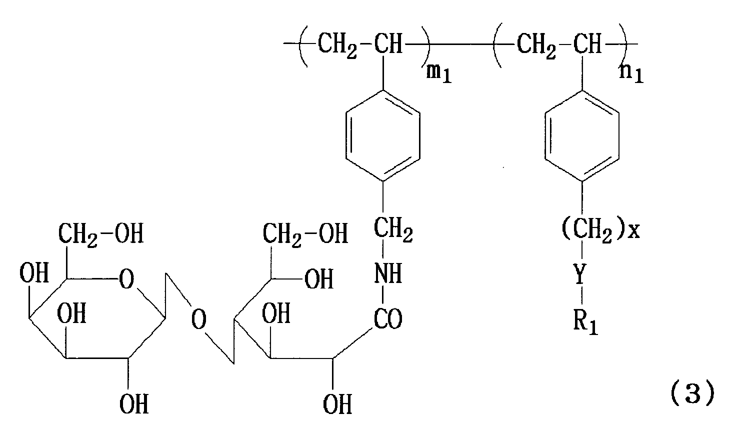 [wherein
m₁:n₁ is 9:1 to 1:9;
x is an integer of 0 to 3;
Y is a single bond, -NHCO-, -NHCONH-, -CONH-, -OCONH-, -NHCOO-, -OCO-, -COO-, -CO- or -NH-;
R₁ is a saturated or unsaturated, monocyclic or dicyclic ring of 5 to 10 carbon(s), or a 5-or 6-membered heterocyclic ring containing O, S or N (wherein the monocyclic or dicyclic ring, or the heterocyclic ring may be unsubstituted or substituted with one or more group(s) selected from the group consisting of halogen, hydroxy, amino, thiol, cyano, cyclopentyl, cyclohexyl, phenyl and acetyl), or alkyl of 1 to 20 carbon(s) (wherein the alkyl may be interrupted with O, S or NH, and may be substituted with one or more group(s) selected from the group consisting of halogen, hydroxy, amino, thiol, cyano, cyclopentyl, cyclohexyl, phenyl and acetyl)],
a copolymer of formula (4): 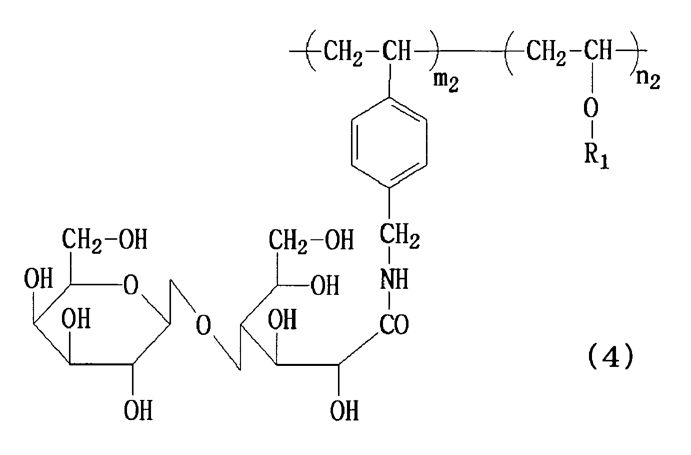 (wherein m₂:n₂ = 9:1 to 1:9; R₁ is as defined for formula (3)); and
a copolymer of formula (5): 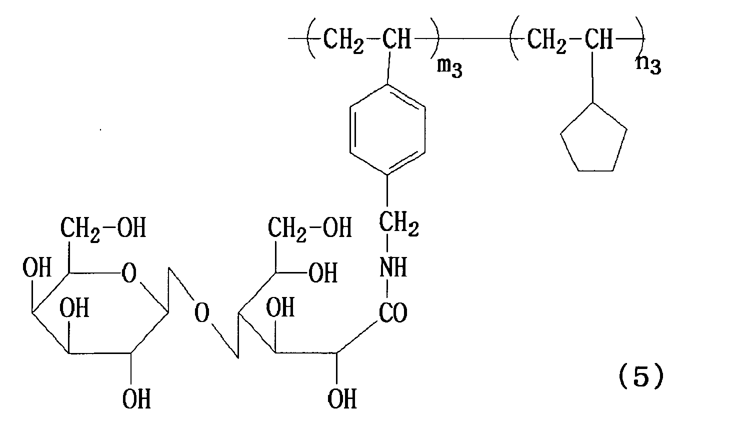 (wherein m₃:n₃ = 9:1 to 1:9).

3. The moisturizer according to claim 2, wherein Y is NHCO- or -CONH-, and R₁ is alkyl of 1 to 20 carbon(s) (wherein the alkyl is substituted with halogen, hydroxy or cyclohexyl).

4. The moisturizer according to any one of claims 1 to 3, wherein the content of the copolymer is 0.0001 to 5% by weight.

5. A cosmetic comprising the moisturizer according to any one of claims 1 to 3, and a polysaccharide and/or a protein.

6. The cosmetic according to claim 5, wherein the polysaccharide is chondroitin.

7. The cosmetic according to claim 5, wherein the polysaccharide is hyaluronic acid.

8. The cosmetic according to claim 5, wherein the protein is collagen or low-molecular-weight collagen.
